# EUROPEAN PATENT SPECIFICATION

(11) **EP 4 137 820 B1**
(45) Date of publication and mention of the grant of the patent: **30.04.2025**
(21) Application number: 21382767.8
(22) Date of filing: 19.08.2021
(51) Int. Cl.: G01N 35/00, G01N 35/04, G16H 10/40

(54) **MASKING OF LABORATORY DEVICES AND ROUTING OF TEST SAMPLE CONTAINERS IN A ABORATORY SYSTEM**
MASKIERUNG VON LABORVORRICHTUNGEN UND ROUTING VON TESTPROBENBEHÄLTERN IN EINEM LABORSYSTEM
MASQUAGE D'APPAREILS DE LABORATOIRE ET ACHEMINEMENT DE RÉCIPIENTS D'ÉCHANTILLONS D'ESSAI DANS UN SYSTÈME DE LABORATOIRE

(43) Date of publication of application: 22.02.2023
(73) Proprietor: F. Hoffmann-La Roche AG, 4070 Basel (CH); Roche Diagnostics GmbH, 68305 Mannheim (DE)
(72) Inventor: FERNÁNDEZ GULIAS, Carlos, 08174 Sant Cugat del Vallès (ES); LEÓN PUJOLÀ, Vanesa, 08174 Sant Cugat del Vallès (ES); PELLITERO CORNET, Laura, 08174 Sant Cugat del Vallès (ES); ROURA BRUN, Pau, 08174 Sant Cugat del Vallès (ES); SMIT, Werner, 6343 Rotkreuz (CH); TAN, Chye Yin Priscillia, 6343 Rotkreuz (CH); URRUTIA JOU, Gemma, 08174 Sant Cugat del Vallès (ES); VON HOPFFGARTEN, Moritz, 68165 Mannheim (DE)
(74) Representative: Herren, Barbara

(56) References cited:
- EP-A1- 2 919 015
- WO-A1-2019/113296
- WO-A2-2013/070755

## Description

### Technical Field

The present disclosure generally relates to maintaining test sample container carrier traffic flow in periods of disruption and/or high use in a laboratory system.

### Background

In a typical laboratory, there currently is not an efficient way to perform overall maintenance in transportation systems in large laboratories that process test sample continuously without requiring a full laboratory stoppage.

Document US Pat. 9,470,702 discloses a method of performing maintenance on an analyzer that include determining a maintenance operation to perform, automatically selecting a maintenance carrier to perform the maintenance operation in response to detecting an error or at a prescheduled time, and under control of a processor, automatically deploying the maintenance carrier onto a track when the affected stations are prepared and not in use when the maintenance carrier arrives.

Document US Pub. 2015/0273691 discloses a system where certain maintenance processes, such as, routine track cleanings, can be scheduled such as, for example, at the end of each shift of an operator. An operator can request when a maintenance operation occurs. The system can determine the moment when maintenance is appropriate based on current conditions with the analyzer and allows maintenance to be performed without interfering with normal sample analysis and operations of the analyzer.

Document US Pat. 10,094,843 discloses a laboratory sample distribution system comprising a transport plane and a cleaning device for cleaning the transport plane. The cleaning device is adapted to automatically clean the transport plane in a way that is similar to the way sample container carriers move on the transport plane.

Document WO 2013/070755 A2 discloses a method for loading specimen containers into centrifuge adapters.

Therefore, there is a need to technically prevent a complete laboratory system stoppage while providing maintenance to the laboratory analyzers and/or transportation systems while still maintaining peak performance of the laboratory system. In addition, there is also a need to ensure the proper priorization of the test sample container carrier during periods of high laboratory workloads without losing the flexibility that a laboratory middleware test sample workflow engine provides.

### Summary

It is an object of the present disclosure to provide a method of routing test sample container carrier during periods of laboratory disruption in a laboratory system by masking certain laboratory analyzers and/or other target devices such that sample containers are not allowed to exit the laboratory analyzers and/or other target devices in order to create the necessary time window for performing crucial laboratory maintenance tasks.

There are several diverse reasons why it might be necessary to prevent sample test containers from exiting a retrievable device such as, for example, laboratory analyzer, buffer, and/or archival device.

One reason is to stop the laboratory system from transporting sample test containers to provide maintenance tasks such as, for example, cleaning the transportation surface of the transportation system. Additional maintenance tasks may include replacing a transportation tile or plate when a magnetic transportation system is used or replacing a belt when using a conveyor belt transportation system. Additionally, when a transportation plate is broken or damaged, it is important to ensure that no sample test containers are on or move on that broken or damaged transportation plate.

Another reason to stop the laboratory system from transporting test sample container carriers is when the laboratory system is about to finish the last shift of the day. In this case, the test sample container carriers that are not already in an archival target location should still be processed by the laboratory system but no additional test sample container carriers should be loaded into the laboratory system for processing.

Another reason to prevent test sample container carriers from exiting a retrievable device is to reduce the workload on the transportation system. For example, when a large batch of test sample container carriers with a high processing priority, e.g., STAT status, arrives at the laboratory system, the analytical devices of the laboratory system need to be available to process those high processing priority test sample container carriers first.

The present invention relates to a method of routing test sample container carriers during periods of laboratory disruption in a laboratory system, as defined in claim 1, and to a laboratory system, as defined in claim 11. Preferred features of the invention are set out in the dependent claims.

The advantages of the present disclosure comprise a gain in efficiency of the overall laboratory and better management of test sample flow in periods of high workloads on the laboratory transportation system and the laboratory system. Upon competition of the maintenance event (or any other event requiring the retrieval masking of a laboratory device), a recalculation of the workflow by the laboratory middleware can occur in order to efficiently restart the processing of the test samples that had remained in the laboratory device during the masking of that laboratory device.

According to the present disclosure, laboratory devices can be masked by the laboratory system so that test sample container carriers cannot be retrieved from the laboratory devices, i.e., test sample container carriers cannot leave the laboratory device. When a laboratory device is masked, the laboratory system will deem that any test sample container carriers inside the device cannot be retrieved. When a laboratory device is unmasked, the laboratory system via the laboratory middleware will recalculate the test sample workflow. This means the laboratory system will determine which test samples in the test sample container carriers in the previously masked laboratory device need to have further processing and will request that those test sample container carriers be retrieved for further processing.

The laboratory system can mask and unmask specific laboratory devices in the plurality of laboratory devices connect to or integrated with the transportation system. By that, it can be possible to continue retrieving and processing test sample container carriers on the non-effected portions of the transportation system.

The retrieval masking of certain laboratory devices can be limited to a specific subset of test sample container carriers. For example,
a) Test sample container carriers with normal priority may not be retrieved from masked laboratory devices while test sample container carriers with STAT priority may be retrieved if required by the laboratory workflow.
b) Test sample container carriers resident in a temporary buffer may not be retrieved if their intended target is an archival device, while those test sample container carriers in the temporary buffer with open test requests for the test samples in the test sample container carriers whose intended target is a laboratory analyzer may be retrieved from the temporary buffer for further processing.
c) Test sample container carriers whose intended target is a laboratory analyzer with expected high number of STAT test samples in the near future will not be retrieved while test sample container carriers whose intended target is a laboratory analyzer with free capacity may be retrieved for further processing.

In addition, retrieval masking can also be applied to specific test sample container carriers at a specific laboratory device. For example, test sample container carriers for which a specific test result was received that had recently visited a laboratory analyzer or that needs to be cooled down for a certain time may not be retrieved from an archival laboratory device.

Retrieval masking of a laboratory device can be enabled/disabled automatically via laboratory configuration rules of the laboratory middleware that can be triggered by internal or external events. Internal triggers can be, for example, a daily maintenance job schedule; daily peak workload time is approaching/ending; daily end of normal business time or the daily beginning of normal business is approaching; an unusually high/low number of new incoming test sample container carriers is registered; and the like. External triggers can be, for example, laboratory instrument or module status messages from the laboratory middleware reporting the availability/unavailability of certain laboratory instruments or modules.

Alternatively, the retrieval masking of a laboratory device can be enabled/disabled manually through a laboratory operator interface with the laboratory middleware of the laboratory system.

In addition, the activation/deactivation of retrieval masking of a laboratory device can be triggered immediately or can be applied with a preconfigured delay such as, for example, at the end of the day or shift.

### Brief Description of the Several Views of the Drawings

The following detailed description of specific embodiments of the present disclosure can be best understood when read in conjunction with the following drawings, where like structure is indicated with like reference numerals and in which:
Fig. 1 illustrates a typical laboratory system setup according to an embodiment of the present disclosure.
Fig. 2 illustrates a flowchart of a method for routing of test sample container carriers while in a masked laboratory device in a laboratory system according to an embodiment of the present disclosure.
Fig. 3 illustrates a flowchart of a method for routing test samples during periods of a blocked transportation connection point in a laboratory system according to an embodiment of the present disclosure.
Fig. 4 illustrates a flowchart of a method for routing test samples at the end of a shift/day in a laboratory system according to an embodiment of the present disclosure.
Fig. 5 illustrates a flowchart of a method for routing test samples during periods of transportation system maintenance in a laboratory system according to an embodiment of the present disclosure.
Fig. 6 illustrates a flowchart of a method for routing test samples during periods of peak sample/STAT sample loading in a laboratory system according to an embodiment of the present disclosure.
Fig. 7 illustrates a flowchart of a method for routing test samples during periods of urgent laboratory analyzer maintenance in a laboratory system according to an embodiment of the present disclosure.

### Detailed Description

In the following detailed description of the embodiments, reference is made to the accompanying drawings that form a part hereof, and in which are shown by way of illustration, and not by way of limitation, specific embodiments in which the disclosure may be practiced. It is to be understood that other embodiments may be utilized and that logical, mechanical and electrical changes may be made without departing from the spirit and scope of the present disclosure.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

The use of the 'a' or 'an' can be employed to describe elements and components of the embodiments herein. This is done merely for convenience and to give a general sense of the inventive concepts. This description should be read to include one or at least one and the singular includes the plural unless it is obvious that it is meant otherwise.

The term "test sample" as used herein can be a broad term and can be given its ordinary and customary meaning to a person of ordinary skill in the art and may not be limited to a special or customized meaning. The term specifically may refer, without limitation, to an aliquot of a substance such as a chemical or biological compound. Specifically, the test sample may be or may comprise at least one biological specimen, such as one or more of: blood; blood serum; blood plasma; urine; saliva. Additionally, or alternatively, the test sample may be or may comprise a chemical substance or compound and/or a reagent. The test sample may specifically be a liquid test sample, such as an aliquot of a fluid substance of the chemical or biological compound. For example, the liquid test sample may be or may comprise at least one pure liquid, such as a liquid substance and/or a solution containing one or more liquid substances, comprising the at least one chemical and/or the biological substance. As another example, the liquid test sample may be or may comprise a liquid mixture, such as a suspension, an emulsion and/or a dispersion of one or more chemical and/or biological substances. However, other, in particular non-liquid test samples can be possible. For example, the container may be a reagent container. Other test sample types may be, for example, tissue, homogenized material, calibration or monitoring container-like devices may be the handling subject.

The term "test sample container" as used herein can be a broad term and can be given its ordinary and customary meaning to a person of ordinary skill in the art and may not be limited to a special or customized meaning. The term specifically may refer, without limitation, to a receptacle, which can be configured for one or more of containing, storing and/or transporting a test sample, specifically, a liquid test sample. Further, the test sample container may be configured for being handled in a test sample handling system. Specifically, the test sample container may be used in the field of medical and/or chemical laboratories. For example, the test sample container may be selected from the group comprising of: a vessel; a vial; a syringe; a cartridge; an ampoule; or a container. For example, the test sample container may comprise a test sample container body for containing the test sample and a test sample container closure, such as a cap for sealing the test sample container. In the following, without restricting further possibilities, the option of a test sample tube will be described, wherein the test sample tube, as an example, may be positioned in a test sample holder, with an open end pointing upwards.

The term ' test sample container carrier' as used herein can refer to any kind of holder configured to receive one or more test sample containers and configured to be used for transporting test sample container(s). Test sample container carriers may be of two major types, single holders and sample racks.

A 'single holder' can be a type of test sample container carrier configured to receive and transport a single test sample container. Typically, a single holder can be provided as a puck, i.e., a flat cylindrical object with an opening to receive and retain a single test sample container.

A 'sample rack' can be a type of test sample container carrier, typically made of plastics and/or metal, adapted for receiving, holding and transporting a plurality of test sample container, e.g., five or more test sample container e.g., disposed in one or more rows. Apertures, windows or slits may be present to enable visual or optical inspection or reading of the test sample container or of the test samples in the test sample container or of a label, such as a barcode, present on the test sample container held in the sample rack.

The term 'laboratory instrument' or "laboratory device" as used herein can encompass any apparatus or apparatus component operable to execute and/or cause the execution of one or more processing steps /workflow steps on one or more biological samples and/or one or more reagents. The expression 'processing steps' thereby can refer to physically executed processing steps such as centrifugation, aliquotation, sample analysis and the like. The term 'instrument' can cover pre-analytical instruments, post-analytical instruments, analytical instruments and laboratory middleware.

The term 'laboratory middleware' as used in the present description can refer to any physical or virtual processing device configurable to control a laboratory instrument/device or system comprising one or more laboratory instruments/devices in a way that workflow(s) and workflow step(s) can be conducted by the laboratory instrument/system. The laboratory middleware may, for example, instruct the laboratory instrument/system to conduct pre-analytical, post analytical and analytical workflow(s)/ workflow step(s). The laboratory middleware may receive information from a data management unit regarding which steps need to be performed with a certain test sample. In some embodiments, the laboratory middleware can be integral with a data management unit, can be comprised by a server computer and/or be part of one laboratory instrument or even distributed across multiple instruments of the laboratory system. The laboratory middleware may, for instance, be embodied as a programmable logic controller running a computer-readable program provided with instructions to perform operations.

A 'data storage unit' or 'database' can be a computing unit for storing and managing data such as a memory, hard disk or cloud storage. This may involve data relating to biological/medical test sample(s) to be processed by the automated system. The data management unit may be connected to an LIS (laboratory information system) and/or an HIS (hospital information system). The data management unit can be a unit within or co-located with a laboratory instrument. It may be part of the laboratory middleware. Alternatively, the database may be a unit remotely located. For instance, it may be embodied in a computer connected via a communication network.

The term 'communication network' as used herein can encompass any type of wireless network, such as a WiFi^{™}, GSM^{™}, UMTS or other wireless digital network or a cable based network, such as Ethernet^{™} or the like. In particular, the communication network can implement the Internet protocol (IP). For example, the communication network can comprise a combination of cable-based and wireless networks.

The term 'remote system' or 'server' as used herein can encompass any physical machine or virtual machine having a physical or virtual processor, capable of receiving; processing and sending data. A server can run on any computer including dedicated computers, which individually can also often be referred to as 'the server' or shared resources such as virtual servers. In many cases, a computer can provide several services and have several servers running. Therefore, the term server may encompass any computerized device that shares a resource with one or more client processes. Furthermore, the terms 'remote system' or 'server' can encompass a data transmission and processing system distributed over a data network (such as a cloud environment).

The term "transportation system" as used herein can be a broad term and can be given its ordinary and customary meaning to a person of ordinary skill in the art and may not be limited to a special or customized meaning. The term specifically may refer, without limitation, to an arbitrary system, which can be configured for moving and/or transporting and/or transferring and/or carrying objects from one position to another. Specifically, the transportation system may be configured for moving the plurality of test sample container carriers through the test sample transportation system such as from a laboratory loading device to another laboratory device of the test sample transportation system. The other laboratory device may be an analysis station. As an example, the transportation system may comprise at least one transport element selected from the group comprising of: a conveyor, such as a belt conveyor or a chain conveyor, or a vehicle system, such as an electronic vehicle system. The test sample transportation system may be or may comprise a multilane transportation system having a plurality of transport elements. The test sample transportation system may be or may comprise a plurality of parallel transport elements. The transport devices may be arranged in a common plane and/or in different planes such as on top of each other.

The term "moving" the plurality of test sample container carriers as used herein, can be a broad term and can be given its ordinary and customary meaning to a person of ordinary skill in the art and may not be limited to a special or customized meaning. The term specifically may refer, without limitation, to an action of transporting and/or transferring and/or carrying the test sample container carriers by using the test sample transportation system. Specifically, the test sample transportation system may be configured for moving the test sample container carriers individually. For example, each of the test sample container carriers may be moved in at least one individual direction, specifically, independently from each other. For example, the movement of the test sample container carriers may be a one-dimensional movement in one direction along the test sample transportation system. As another example, the movement of the test sample container carriers may be a two-dimensional movement in two directions along the test sample transportation system. Additionally, or alternatively, the test sample container carriers may be moved in a third direction by the test sample transportation system by passing a difference in height of the test sample handling system. Further, the test sample transportation system may be configured for moving the plurality of test sample container carriers in a bidirectional manner.

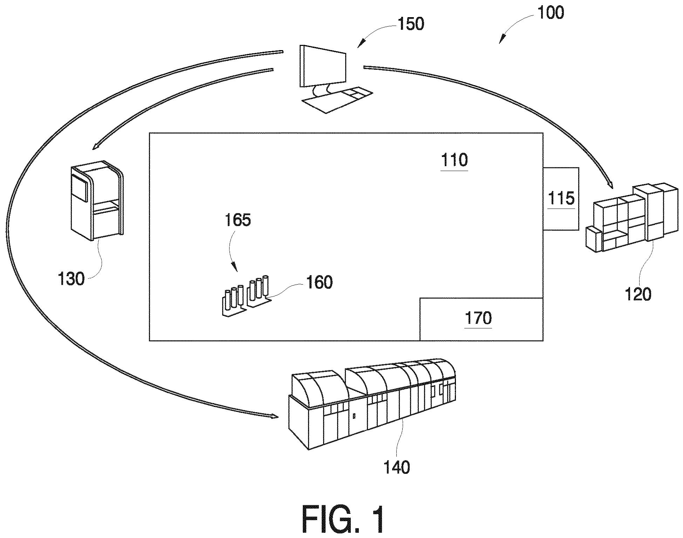

Referring initially to Fig. 1, Fig. 1 illustrates a typical laboratory system setup 100. In a typical laboratory system 100, a plurality of different laboratory devices such as, for example, such as pre-analytical laboratory devices 130, analytical laboratory devices 115, 140, and post-analytical laboratory devices 120 can be connected together via a transportation system 110. A pre-analytical laboratory device 130 can usually be used for the preliminary processing of test samples 165 or test sample container carriers 160. An analytical laboratory device 140 can be designed, for example, to use a test sample or part of the test sample and a test reagent in order to produce a measurable signal, based on which it is possible to determine whether an analyte is present, and if desired in what concentration. A post-analytical laboratory device 120 can be used for the post-processing of test samples or test sample vessels like the archiving of test samples or test sample container carriers.

The transportation system **110** can be used to transport test sample container carriers 160 comprises test samples between the plurality of different laboratory devices 120, 130, 140. Control of the movement of the test sample container carriers 160 between the plurality of laboratory devices 120, 130, 140 can be managed by a laboratory middleware 150. The laboratory middleware 150 can communicate with the plurality of laboratory devices 120, 130, 140 as well as, the transportation system 110.

**In** one embodiment, during the operation of the laboratory system 100, a temporary buffer 170, i.e., an add-on buffer 170, may be formed on the transportation system 110. The temporary buffer 170 can receive and house several test sample container carriers 160 during periods when the analytical laboratory devices 140 may be at capacity and, therefore, can no longer accept test samples. The temporary buffer 170 can serve as place to house the test sample container carriers 160 in order to prevent logjams and bottleneck traffic flow issues on the transportation system 110.

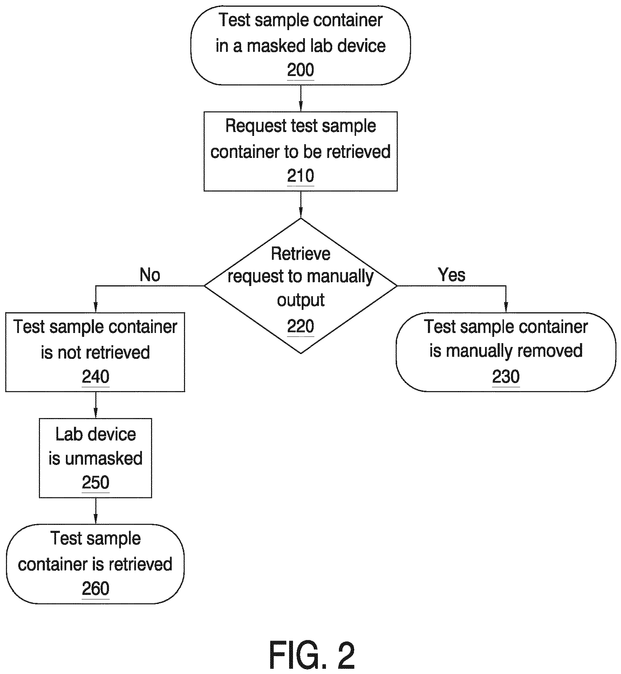

Fig. 2 illustrates a flowchart of a method for routing of test sample container carriers 160 while a laboratory device in a laboratory system 100 is masked. In step 200, a test sample container carrier 160 can be in a typically retrievable laboratory device such as, for example, post-analytical station 120 such as, for example, a refrigerator. However, the retrievable laboratory device 120 can be masked by the laboratory middleware 150 of the laboratory system 100 so that the test sample container carrier 160 cannot leave the laboratory device. The laboratory device 120 can be masked for several reasons, which will be described in more detail in the following.

In step 210, a request can be made by the laboratory middleware 150 of the laboratory system 100 to retrieve one of the test sample container carriers 160 from the masked laboratory device 120. In one embodiment, since the laboratory device 120 is masked, the test sample container carrier 160 cannot be retrieved. In another embodiment, the laboratory middleware 150 of the laboratory system 100 can determine if the target laboratory device 140 for which the test samples 165 in the test sample container carrier 160 is needed is also masked. If the target laboratory device 140 is not masked, the test sample container carrier 160 may be retrieved and sent to the unmasked target device 140.

In step 220, the laboratory middleware 150 of the laboratory system 100 can then determine if manual retrieval of the test sample container carrier 160 from the masked laboratory device 120 was requested by a laboratory operator. If manual retrieval was requested, the test sample container carrier 160 can be retrieved, in step 230, independently of the masking status of the laboratory device 120. If manual retrieval was not requested, the test sample container carrier 160, in step 240, is not retrieved.

In step 250, the laboratory device 120 is unmasked. After unmasking, the workflow for the test sample container carriers 160 in the previously masked laboratory device 120 can be recalculated by the laboratory middleware 150. If it is determined that one of the test sample container carriers 160 needs to be retrieved because, for example, that test sample container carrier 160 has outstanding open test results for the test samples 165, time has expired for that test sample container carrier 160 at that laboratory device 120, the forced retrieval was unsuccessful, and the like, that test sample container carrier 160 is retrieved in step 260.

### Examples

### Example 1: A blocked transportation connection to a target device

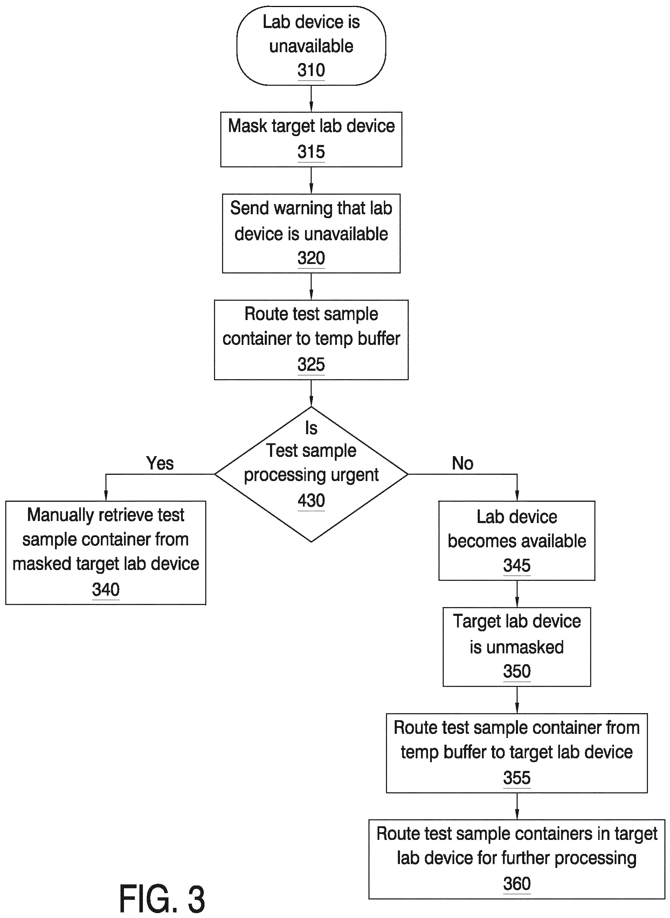

In this example, a connection point 115 such as, for example, a bidirectional reformatter, between the transportation system 110 and a target laboratory device 120 such as, for example, an archival laboratory device 120 such as, for example, a refrigerator becomes unavailable. Fig. 3 illustrates a flowchart of this method for routing test samples during periods of a blocked transportation connection point in a laboratory system.

In step 310, the laboratory middleware 150 receives a status message from a laboratory analytical device 140 indicating that transportation connection point 115 is not available. This message than triggers, in step 315, the masking of the target device 120 for sorting, i.e., there will no attempt to route test sample container carriers 160 from the transportation system 110 to the target laboratory device 120 through the unavailable connection point 115, as well as for retrieval, i.e., there will be no attempt to retrieve test sample container carriers 160 from the target laboratory device 120 through the unavailable connection point 115.

At the same time as the target laboratory device 120 is masked, a laboratory operator may receive a warning about the unavailability of the connection point 115, in step 320. The laboratory operator can be warned in several ways. For example, a visual indication on the connection point 115 itself such as, for example, a red error or yellow warning light can flash depending on the reason of the connection point's 115 unavailability; a notification message may appear on the graphic user interface (GUI) of the control unit of the target laboratory device 120; and/or a notification message may appear in the notification section of the laboratory middleware 150 GUI.

The test sample container carriers 160 that were originally being routed to the target laboratory device 120 will, in step 325, now be routed to/or remain in a temporary buffer or add-on buffer (AOB) 170 section of the transportation system 110. The routing of the test sample container carriers 160 can be controlled by the laboratory middleware 150 by requesting that the temporary buffer 170 be used in place of the target laboratory device 120 or by not sending a sample retrieval request to those test sample container carriers 160 already in the temporary buffer 170.

Without this functionality, the laboratory middleware 150 typically would have requested that the test sample container carriers 160 be routed to the target laboratory device 120. Since the connection point 115 was unavailable, the laboratory middleware 150 would have had to send the test sample container carriers 160 to alternative destinations since the requested laboratory target 120 was not available. In the worst case, this could result in the loss of test sample container carrier tracking information when the laboratory middleware 150 is unable to interpret the misalignment between expected and actual test sample container carrier 160 routing. Additionally, laboratory middleware 150 functionality that could be used to manage workload balancing across multiple target laboratory devices 140, 120 on the transportation system 110 could be comprised since test sample container carriers 160 could be unintentionally routed to unexpected destinations.

Additionally, test sample container carriers 160 already located in the target laboratory device 120 will remain in the target laboratory device 120 if additional testing had been requested of those test sample container carriers 160. The laboratory middleware 150 will simply not send sample retrieval requests for test sample container carriers 160 currently in a masked target laboratory device 120.

In one embodiment, if it is determined, in step 335, that the processing of the test sample container carriers 160 is urgent i.e., the test sample container carrier 160 contains STAT test samples, the laboratory operator can request manual retrieval of that test sample container carrier 160 from the target laboratory device 120 via the user control interface of the laboratory middleware 150, in step 340, and will be able to retrieve and manually process test samples 165 in that test sample container carrier 160.

Without this functionality, the laboratory middleware 150 would have requested that the test sample container carriers 160 be retrieved from the target laboratory device 120. Without this functionality, the target laboratory device 120 would be unaware of the unavailable connection point 115. With the unavailable connection point 115, test sample container carriers 160 would be retrieved from the target laboratory device 120 but would not be able to be processed any further. This would result in the sample test containers 160 remaining in the area of the connection point 115 and transportation system 110, an area that is not temperature or humidity controlled, until the connection point 115 becomes functional. At this location, it will also be more difficult for the laboratory operator to manually access important test sample container carriers 160 for further processing. If the test sample container carrier 160 were in the area of the connection point 115, the laboratory operator would have to search for test sample container carrier 160 in the group of retrieved and locked test sample container carriers 160. Instead, if the test sample container carriers 160 remain the target laboratory device 120, the laboratory operator can simply request manual retrieval of that test sample container carrier 160 from the target laboratory device 120 via the user control interface of the laboratory middleware 150.

The laboratory operator can determine and fix the issue with the connection point 115 and the connection point 115 becomes available again in step 345. The laboratory middleware 150 will then receive a status message that the connection point 115 is now operational. This message then triggers the laboratory middleware 115 to unmask the target laboratory device 120 for both sorting and retrieval in step 350. After unmasking, the workflow for the test sample container carriers 160 in the previously masked laboratory device 120 can be recalculated by the laboratory middleware 150.

In step 355, test sample container carriers 160 that had been routed to the temporary buffer 170 while the target laboratory device 120 was masked can now be retrieved from the temporary buffer 170 and sent to the target laboratory device 120. This process is controlled by the laboratory middleware 150 by sending test sample retrieval request messages to the transportation system 110.

Additionally, in step 360, the test samples 165 in the test sample container carriers 160 with outstanding test requests that were in the target laboratory device 120 while the target laboratory device 120 was masked can now be retrieved from the target laboratory device 120 for further processing.

### Example 2: End of a shift/End of a day

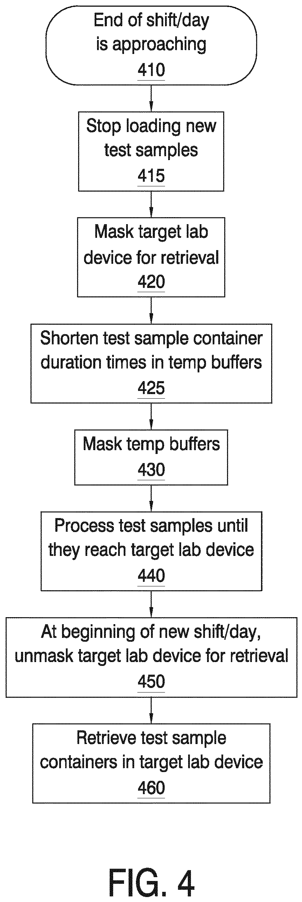

In this example, the end of a normal laboratory routine operation is approaching and the laboratory operator wants to reduce and ultimately stop the test sample processing on the laboratory system 100 and its connected laboratory analytical devices 140. Fig. 4 illustrates a flowchart of a method for routing test sample container carriers at the end of a shift/day in a laboratory system 100.

At the end of the day/shift, the laboratory operator, in step 415, can stop loading test samples container carriers into the input modules of the pre-analytic devices 130 of the transportation system **110** of the laboratory system 100. Instead, all incoming test samples container carriers 160 can be manually stored in a post-analytical device 120 such as, for example, an archival device such as, for example, a refrigerator for processing on the next day/shift.

**In** step 420, the laboratory operator accesses the laboratory middleware 150 routine client to mask the archival target device 120 for retrieval, i.e., no test sample container carriers 160 will be able to leave the archival target device 120. **In** another embodiment, the masking of the archival target device 120 can happen automatically based on the configuration of the rule engine of the laboratory middleware 150 such as, for example, the retrieval masking can occur based on the time of day.

In one embodiment, the masking of the archival target device 120 for retrieval can be applied to only test sample container carriers 160 containing test samples 165 with routine priority. This way, the STAT test samples 165 can still be processed the same way during normal operating times.

In one embodiment, in step 425, the temporary buffer targets 170 can reduce the test sample container carrier 160 buffer duration times, i.e., the amount of time a test sample container carrier 160 can remain in the temporary buffer 170 assigned to the test sample container carriers 160 so that the test sample container carriers 160 are retrieved from the temporary buffer 170 and routed to the archival target device 120 sooner. This reduction in time can be configured to happen automatically based on the time of day by the laboratory middleware 150.

In one embodiment, in step 430, the laboratory operator can access the laboratory middleware 150 routine client to mask the temporary buffer targets 170 for distribution so that the test sample container carriers 160 are routed directly to the archival target device 120 once all open test requests are processed for those test sample container carriers 160. In another embodiment, the masking of the temporary buffer target device 170 can happen automatically based on the configuration of the rule engine of the laboratory middleware 150 such as, for example, based on the time of day.

In step 440, all test sample container carriers 160 still located on the laboratory transportation system 110 or in the connected laboratory analytic devices 140 will continue to be processed as normal. However, once the test sample container carriers 160 reach the archival target device 120, the test sample container carriers 160 will not be retrieved for any additional processing.

Then, when routine operation resumes the next day/shift, the laboratory operator, in step 450, accesses the laboratory middleware 150 routine client and unmasks the archival target devices 120 for retrieval. In another embodiment, the unmasking of the archival target device 120 can happen automatically based on the configuration of the rule engine of the laboratory middleware 150 such as, for example, based on the time of day. After unmasking, the workflow for the test sample container carriers 160 in the previously masked archival laboratory device 120 can be recalculated by the laboratory middleware 150.

In step 460, the test sample container carriers 160 that currently are in the archival target device 120 and that have open test requests for the test samples 165 in the test sample container carriers 160 will be retrieved by the laboratory system 100 to the transportation system 110. This occurs by the laboratory middleware 150 sending retrieval requests to the archival target device 120.

Without this functionality, the reduction of workload into the laboratory system 100 may only be achieved by switching off the laboratory analytic devices 140 and collecting the test sample container carriers 160 that have already been retrieved from the archival target device 120. This would result in test samples 165 being in non-temperature and humidity controlled environments. Additionally, without this functionality, the automated handling of STAT test samples 165 different from routine handling of test sample container carriers 160 would not be allowed.

### Example 3: Maintenance of a limited part of the transportation system

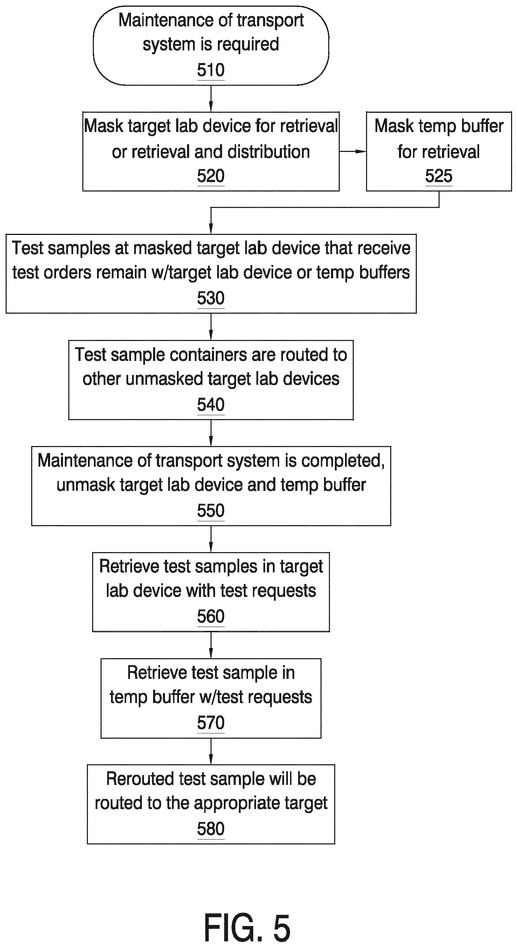

In this example, maintenance of part of the transportation system 110 is necessary while the rest of the transportation system 110 remains functional is illustrated. Fig. 5 illustrates a flowchart of a method for routing test sample container carriers 160 during periods of transportation system 110 maintenance in a laboratory system 100.

In step 510, it is determined that maintenance of part of the transportation system 110 is necessary since that part of the transportation system 110 is crucial for the routing of retrieved test sample container carriers 160 from an archive target device 120 or a test sample temporary buffer 170 to the laboratory analytic devices 140 or that routing of test sample container carriers 160 around the section of the transportation system 110 needing maintenance results in traffic jams and a locked transportation system 110.

In step 520, the target laboratory device 120 is masked for retrieval or for both retrieval and distribution depending on the impact to the transportation system 110.

In step 525, temporary buffers 170 are also masked for retrieval or for both retrieval and distribution depending on the impact to the transportation system 110.

In step 530, test sample container carriers 160 already at the target laboratory device 120 or in the temporary buffers 170 but receive addition al test orders for the test samples 165 in the test sample container carriers 160 will remain at the target laboratory device 120 or temporary buffer 170. This allows the workload of the laboratory system 100 to be reduced during maintenance of the transportation system 110.

In one embodiment, if the target laboratory device 120 is masked for distribution of test sample container carriers 160, test sample container carriers 160 usually routed to the target laboratory device 120 will now be routed to alternative targets in step 540. This will result in redirecting test sample container carrier 160 flow away from the affected area of the transportation system 110.

Depending on the nature of the laboratory system 100, the nature of the root cause of the maintenance issue, and the exact location of maintenance issue on the transportation system 110, the masking can either be triggered automatically through the processing of module availability messaging by the laboratory middleware 150 or manually by the laboratory operator through access via the routine client of the laboratory middleware 150.

Once the maintenance is completed, the affected target laboratory devices 120 and temporary buffers 170 can be unmasked, in step 550, either manually by the laboratory operator or automatically through the processing of module availability messaging of the laboratory middleware 150. After unmasking, the workflow for the test sample container carriers 160 in the previously masked laboratory device 120 can be recalculated by the laboratory middleware 150.

In step 560, the test samples 165 in the test sample container carriers 160 with open test requests that had remained in the previously masked laboratory target device 120 and/or temporary buffer devices 170 can now be retrieved. This process is controlled by the laboratory middleware 150 by sending sample retrieval request messages to the previously masked laboratory target devices 120 and/or temporary buffer devices 170.

In step 570, test sample container carriers 160 that had remained in test sample temporary buffer target 170 even though their storage duration had elapsed while the test sample temporary buffer 170 was masked for retrieval can now be retrieved and routed to their final archival target device 120. This process is controlled by the laboratory middleware 150 by sending sample retrieval request messages to the previously masked temporary buffer devices 170.

In step 580, the test sample container carriers 160 that had been rerouted to alternative targets while their original target laboratory device 120 were masked for distribution can follow different scenarios.
a) If the test sample container carriers 160 were routed to an alternative target laboratory device that was a final archival target laboratory device 120, the test sample container carriers 160 will remain at that archival target laboratory device 120 until additional test requests for the test samples 165 in the test sample container carriers 160 are received or the test samples 165 are fully disposed of.
b) If the test sample container carriers 165 were originally routed to an alternative target laboratory device 120 that was masked for distribution and were routed to a test sample temporary buffer 170 instead, the test sample container carriers 160 will be retrieved from that test sample temporary buffer 170 and routed to the target laboratory device 120.
c) If the test sample container carriers 160 were originally routed to an alternative test sample temporary buffer target 170 that was masked for distribution and were routed to an alternative test sample temporary buffer, the test sample container carriers 160 will remain in the alternative test sample temporary buffer until either an addition test request is received for the test samples 165 in the test sample container carrier 160 and the test sample container carrier 160 is retrieved to go to the appropriate laboratory analytical device 140 or the storage duration for that test sample in the test sample temporary buffer 170 elapses and the test sample container carrier 160 is retrieve to go to a final archival target device 120.
Note that cases a-c are merely examples of a reasonable configuration for the laboratory middleware 150. Different behavior such as, for example, prioritizing the initial target device even if test samples containers 160 had been routed to a target device of similar function, can be configured as well.

Without this functionality, either the entire laboratory system 100 would have to be shut down for maintenance even if that would not be required from the hardware itself or the overloading of the transportation system 110 or parts of the transportation system 110 could not be prevented. Depending on the nature of the transportation system 110, overloading a small part of the transportation system 110 can stop the complete transportation system 110 from working properly.

### Example 4: Peak time for new incoming test samples

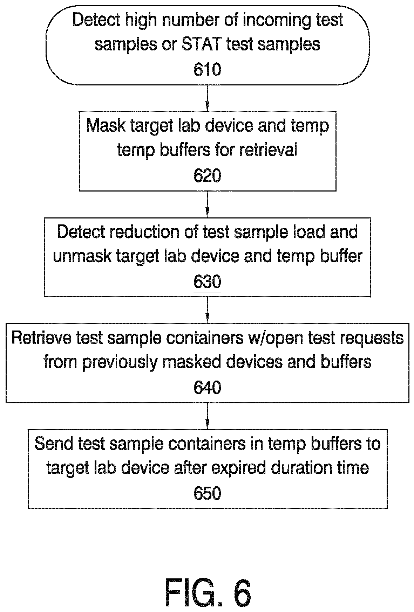

**In** this example, the prevention of the overloading of the laboratory system 100 due to a peak time of new incoming test samples 165 is described. Fig. 6 illustrates a flowchart of a method for routing test sample container carriers 160 during periods of peak sample/STAT sample loading in a laboratory system 100.

**In** step 610, a high number of incoming test sample container carriers 160 or an incoming batch of test sample container carriers 160 from an emergency ward (i.e., STAT test samples 165) is detected by the laboratory middleware 150 of the laboratory system 100. The detection of the test sample container carriers 160 can happen by:
a) By a large number of incoming order messages. However, this may not always be related to an increase of incoming test sample container carriers 160.
b) By a large number of sample identifications on the input stations of the pre-analytical devices 130 of the laboratory system 100.
c) By a reduction of queue or rack availability across the complete transportation system 110 as reported via the respective messages sent by the laboratory system 100 to the laboratory middleware 150
d) By an increase of turnaround time of test samples in the laboratory analytic devices 140 or an increase of the number of timeouts of the test samples 165 in the queue.

In anticipation of the large number of test samples containers 165 or of STAT test sample container carriers, in step 620, the archival target device 120 and/or the temporary buffer targets 170 are masked for retrieval automatically. This masking can help reduce the workload or help prioritize STAT test samples on the laboratory system 110.

When the reduction of the test sample container carriers 160 is detected or the STAT test sample container carriers have been processed, the masked archival laboratory target 120 and temporary buffer targets 170 are unmasked for retrieval in step 630. After unmasking, the workflow for the test sample container carriers 160 in the previously masked laboratory device 120 can be recalculated by the laboratory middleware 150.

In step 640, the test samples 165 in the test sample container carriers 160 with open test requests that had been in the masked archival laboratory device 170 and temporary buffer target 170 will now be retrieved. This process is controlled by the laboratory middleware 150 by sending sample retrieval request messages to the previously masked archival laboratory target 120 and/or temporary buffer devices 170.

In step 650, the test sample container carriers 160 that had remained in test sample temporary buffer targets 170 even though their storage duration had elapsed while the test sample temporary buffer 170 was masked for retrieval can now be retrieved and routed to their final archival target laboratory device 120. This process is controlled by the laboratory middleware 150 by sending sample retrieval request messages to the previously masked temporary buffer devices 170.

Without this functionality, the prevention of a general overloading of the laboratory system 100 would become more difficult in the routine time or would require more complex configuration of the laboratory middleware 150 during the laboratory system 100 set-up time.

### Example 5: Urgent maintenance of an important laboratory analyzer

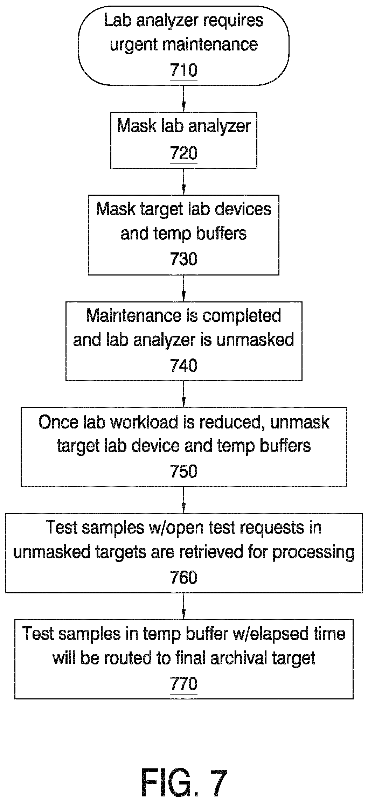

In this example, the prevention of the overloading of alternative analytic laboratory targets due to urgent maintenance of an important laboratory analytic devices is illustrated. Fig. 7 illustrates a flowchart of a method for routing test sample container carriers 160 during periods of urgent laboratory analyzer maintenance in a laboratory system.

In step 710, a laboratory analytical device 140 that usually processes a high fraction of the test sample workload has to be disconnected from the transportation system 110 of the laboratory system 110 to undergo urgent maintenance.

During maintenance, the laboratory analytical device 140 will be masked for distribution in the laboratory middleware 150 in step 720. The laboratory analytical device 140 can be masked either manually by the laboratory operator or by the laboratory middleware 150 sending out a status message.

Additionally, the laboratory operator may foresee a general overloading of the laboratory system 100 and, in step 730, the laboratory operator may mask some, or all, of the test sample temporary buffer targets 170 and/or some, or all, of the archival target laboratory devices 120 for retrieval in order to reduce the test sample workload of the transportation system 110.

In step 740, the maintenance activity on the laboratory analytical device 140 is completed and the laboratory analytical device 140 is unmasked. After unmasking of the laboratory analytical device 140, the workflow for the test sample container carriers 160 in the previously masked laboratory analytical device 140 can be recalculated by the laboratory middleware 150.

Once a reasonable reduction of the workload of the laboratory system 100 is determined, the test sample temporary buffers 170 and the archival target laboratory devices 120, in step 750, are unmasked for retrieval. After unmasking of the test sample temporary buffers 170 and the archival target laboratory devices 120, the workflow for the test sample container carriers 160 in the previously masked laboratory device 120 can be recalculated by the laboratory middleware 150.

In step 760, the test samples 165 in the test sample container carriers 160 with open test requests that had been in the masked archival laboratory device 120 and/or the masked temporary buffer targets 170 will now be retrieved. This process is controlled by the laboratory middleware 150 by sending sample retrieval request messages to the previously masked laboratory target 120 and/or temporary buffer devices 170.

In step 770, the test sample container carriers 160 that had remained in test sample temporary buffer targets 170 even though their storage duration had elapsed while the test sample temporary buffer 170 was masked for retrieval can now be retrieved and routed to their final archival target laboratory device 120. This process is controlled by the laboratory middleware 150 by sending sample retrieval request messages to the previously masked temporary buffer devices 170.

In one embodiment, the retrieval masking process can be applied selectively depending of the type of the transportation system 110, i.e., the process can be applied at the section level if the transportation system 110 allows it such as, for example, if the transportation system 110 comprises belt conveyors.

Further disclosed and proposed is a computer program product including computer-executable instructions for performing the disclosed method in one or more of the embodiments enclosed herein when the program is executed on a computer or computer network. Specifically, the computer program may be stored on a computer-readable data carrier or a server computer. Thus, specifically, one, more than one or even all of method steps as indicated above may be performed by using a computer or a computer network, preferably by using a computer program.

As used herein, a computer program product refers to the program as a tradable product. The product may generally exist in any format, such as in a paper format, or on a computer-readable data carrier on premise or located at a remote location. Specifically, the computer program product may be distributed over a data network (such as a cloud environment). Furthermore, not only the computer program product, but also the execution hardware may be located on premise or in a cloud environment.

Further disclosed and proposed is a computer-readable medium comprising instructions which, when executed by a computer system, cause a laboratory system to perform the method according to one or more of the embodiments disclosed herein.

Further disclosed and proposed is a modulated data signal comprising instructions, which, when executed by a computer system, cause a laboratory system to perform the method according to one or more of the embodiments disclosed herein.

Referring to the computer-implemented aspects of the disclosed method, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Having described the present disclosure in detail and by reference to specific embodiments thereof, it will be apparent that modifications and variations are possible without departing from the scope of the disclosure defined in the appended claims.

## Claims

1. A method of routing test sample container carriers (160) during periods of laboratory disruption in a laboratory system (100) wherein the laboratory system (100) comprises a plurality of laboratory devices (115, 120, 130, 140), at least one buffer (170), a transportation system (110), and a laboratory middleware (150), the method comprising:
determining, by the laboratory middleware (150), whether a laboratory device (115) in the laboratory system (100) is unavailable, wherein the unavailable laboratory device (115) is a connection point between the transportation system (110) and a target laboratory device (120);
masking a, by the laboratory middleware (150), the target laboratory device (120) connected to the unavailable laboratory device (115) so that test sample container carriers (160) are not sent to the target laboratory device (120) and test sample container carriers (160) cannot be retrieved from the target laboratory device (120);
rerouting test sample container carriers (160) originally routed to the target laboratory device (120) before the unavailability of the laboratory device (115) to a buffer (170) located in the laboratory system (100);
calculating a new laboratory system workflow by the laboratory middleware (150) after the unavailable laboratory device (115) becomes available;
unmasking, by the laboratory middleware (150), the target laboratory device (120) after the new laboratory workflow is calculated; retrieving test sample container carriers (160) with outstanding test requests that were in the target laboratory device (120) while the target laboratory (120) was masked from the target laboratory device (120) for further processing; and
retrieving the test sample container carriers (160) from the buffer (170) and sending those test sample container carriers (160) to the target laboratory device (120).

2. The method according to claim 1, wherein the laboratory device (115) is unavailable due to a maintenance issue.

3. The method according to claims 1 and 2, further comprising,
alerting a laboratory operator that the laboratory device (115) is unavailable.

4. The method according to claim 3, wherein the alerting is a visual indicator on the unavailable laboratory device (115).

5. The method according to claim 3, wherein the alerting is a software notification.

6. The method according to any of the preceding claims, further comprising, the laboratory middleware (150) not sending sample retrieval requests for test sample container carriers (160) in the target laboratory device (120) while the target laboratory device (120) is masked.

7. The method according to any of the preceding claims, further comprising, manually accessing test sample container carriers (160) by a laboratory operator in the masked target laboratory device (120) while the target laboratory device (120) is masked.

8. The method according to any of the preceding claims, wherein the target laboratory device (120) is an archival device such as a refrigerator.

9. The method according to any of the preceding claims, wherein the masking and unmasking of the target laboratory device (120) is automatically triggered by laboratory middleware (150) of the laboratory system (100).

10. The method according to any of the preceding claims, further comprising,
rerouting test sample container carriers (160) from the masked target device (120) to other laboratory devices in the plurality of laboratory devices (130, 140) in the laboratory system (100) while the target laboratory device (120) is masked.

11. A laboratory system (100), the laboratory system comprising:
a plurality of laboratory devices (115, 120, 130, 140),
at least one buffer (170),
a transportation system (110), and
a laboratory middleware (150),
**characterized in that** the laboratory system (100) is configured to perform the method of at least one of the preceding claims.

## Patentansprüche

1. Verfahren zum Leiten von Testprobenbehälterträgern (160) während Laborstörungsperioden in einem Laborsystem (100), wobei das Laborsystem (100) eine Vielzahl von Laborvorrichtungen (115, 120, 130, 140), mindestens einen Puffer (170), ein Transportsystem (110) und eine Labor-Middleware (150) umfasst, wobei das Verfahren Folgendes umfasst:
Bestimmen, ob eine Laborvorrichtung (115) in dem Laborsystem (100) nicht zur Verfügung steht, mithilfe der Labor-Middleware (150), wobei die nicht zur Verfügung stehende Laborvorrichtung (115) ein Verbindungspunkt zwischen dem Transportsystem (110) und einer Ziellaborvorrichtung (120) ist;
Maskieren der Ziellaborvorrichtung (120), die mit der nicht zur Verfügung stehenden Laborvorrichtung (115) verbunden ist, mithilfe der Labor-Middleware (150), sodass die Testprobenbehälterträger (160) nicht zu der Ziellaborvorrichtung (120) gesendet werden und Testprobenbehälterträger (160) nicht aus der Ziellaborvorrichtung (120) entnommen werden können;
Umleiten von Testprobenbehälterträgern (160), die vor der Nichtverfügbarkeit der Laborvorrichtung (115) ursprünglich zu der Ziellaborvorrichtung (120) geleitet wurden, zu einem Puffer (170), der sich in dem Laborsystem (100) befindet;
Berechnen eines neuen Laborsystemarbeitsablaufes mithilfe der Labor-Middleware (150), sobald die nicht zur Verfügung stehende Laborvorrichtung (115) zur Verfügung steht;
Demaskieren der Ziellaborvorrichtung (120) mithilfe der Labor-Middleware (150), sobald der neue Laborarbeitsablauf berechnet ist;
Entnehmen von Testprobenbehälterträgern (160) mit ausstehenden Testanfragen, die sich in der Ziellaborvorrichtung (120) befanden, während das Ziellabor (120) maskiert war, aus der Ziellaborvorrichtung (120) zum Weiterverarbeiten; und
Entnehmen der Testprobenbehälterträger (160) aus dem Puffer (170) und Senden dieser Testprobenbehälterträger (160) zu der Ziellaborvorrichtung (120).

2. Verfahren nach Anspruch 1, wobei die Laborvorrichtung (115) aufgrund eines Wartungsproblems nicht zur Verfügung steht.

3. Verfahren nach den Ansprüchen 1 und 2, ferner umfassend das Warnen eines Laborbetreibers, dass die Laborvorrichtung (115) nicht zur Verfügung steht.

4. Verfahren nach Anspruch 3, wobei das Wamen eine visuelle Anzeige auf der nicht zur Verfügung stehenden Laborvorrichtung (115) ist.

5. Verfahren nach Anspruch 3, wobei das Warnen eine Software-Mitteilung ist.

6. Verfahren nach einem der vorstehenden Ansprüche, ferner umfassend, dass die Labor-Middleware (150) keine Probenentnahmeanfragen für Testprobenbehälterträger (160) in der Ziellaborvorrichtung (120) sendet, während die Ziellaborvorrichtung (120) maskiert ist.

7. Verfahren nach einem der vorstehenden Ansprüche, ferner umfassend das manuelle Zugreifen auf Testprobenbehälterträger (160) durch einen Laborbetreiber in der maskierten Ziellaborvorrichtung (120), während die Ziellaborvorrichtung (120) maskiert ist.

8. Verfahren nach einem der vorstehenden Ansprüche, wobei die Ziellaborvorrichtung (120) eine Archivvorrichtung, wie ein Kühlschrank, ist.

9. Verfahren nach einem der vorstehenden Ansprüche, wobei das Maskieren und Demaskieren der Ziellaborvorrichtung (120) von Labor-Middleware (150) des Laborsystems (100) automatisch ausgelöst wird.

10. Verfahren nach einem der vorstehenden Ansprüche, ferner umfassend das Umleiten von Testprobenbehälterträgern (160) aus der maskierten Zielvorrichtung (120) zu anderen Laborvorrichtungen in der Vielzahl von Laborvorrichtungen (130, 140) in dem Laborsystem (100), während die Ziellaborvorrichtung (120) maskiert ist.

11. Laborsystem (100), wobei das Laborsystem Folgendes umfasst:
eine Vielzahl von Laborvorrichtungen (115, 120, 130, 140),
mindestens einen Puffer (170),
ein Transportsystem (110) und
eine Labor-Middleware (150),
**dadurch gekennzeichnet, dass** das Laborsystem (100) dafür ausgebildet ist, das Verfahren nach mindestens einem der vorstehenden Ansprüche auszuführen.

## Revendications

1. Procédé d'acheminement de portes-récipients d'échantillons d'essai (160) pendant des périodes de perturbation de laboratoire dans un système de laboratoire (100) dans lequel le système de laboratoire (100) comprend une pluralité d'appareils de laboratoire (115, 120, 130, 140), au moins un tampon (170), un système de transport (110) et un intergiciel de laboratoire (150), le procédé comprenant :
la détermination, par l'intergiciel de laboratoire (150), si un appareil de laboratoire (115) dans le système de laboratoire (100) est indisponible, l'appareil de laboratoire indisponible (115) étant un point de liaison entre le système de transport (110) et un appareil de laboratoire cible (120) ;
le masquage, par l'intergiciel de laboratoire (150), de l'appareil de laboratoire cible (120) relié à l'appareil de laboratoire indisponible (115) de sorte que les portes-récipients d'échantillons d'essai (160) ne sont pas envoyés à l'appareil de laboratoire cible (120) et les portes-récipients d'échantillons d'essai (160) ne peuvent pas être récupérés de l'appareil de laboratoire cible (120) ;
le réacheminement des portes-récipients de laboratoire d'essais (160) initialement acheminés vers l'appareil de laboratoire cible (120) avant l'indisponibilité de l'appareil de laboratoire (115) vers un tampon (170) situé dans le système de laboratoire (100) ;
le calcul d'un nouveau flux de travail du système de laboratoire par l'intergiciel de laboratoire (150) après que l'appareil de laboratoire indisponible (115) devient disponible ;
le démasquage, par l'intergiciel de laboratoire (150), de l'appareil de laboratoire cible (120) après que le nouveau flux de travail de laboratoire est calculé ;
la récupération des portes-récipients d'échantillons d'essai (160) avec des requêtes d'essai en attente qui étaient dans l'appareil de laboratoire cible (120) pendant que le laboratoire cible (120) était masqué de l'appareil de laboratoire cible (120) pour un traitement ultérieur ; et
la récupération des portes-récipients d'échantillons d'essai (160) du tampon (170) et l'envoi de ces portes-récipients d'échantillons d'essai (160) à l'appareil de laboratoire cible (120).

2. Procédé selon la revendication 1, dans lequel l'appareil de laboratoire (115) est indisponible en raison d'un problème de maintenance.

3. Procédé selon les revendications 1 et 2, comprenant en outre,
l'alerte d'un opérateur de laboratoire sur le fait que l'appareil de laboratoire (115) est indisponible.

4. Procédé selon la revendication 3, dans lequel l'alerte est un indicateur visuel sur l'appareil de laboratoire indisponible (115).

5. Procédé selon la revendication 3, dans lequel l'alerte est une notification logicielle.

6. Procédé selon l'une quelconque des revendications précédentes, comprenant en outre, l'intergiciel de laboratoire (150) n'envoyant pas de requêtes de récupération d'échantillons pour les portes-récipients d'échantillons d'essai (160) dans l'appareil de laboratoire cible (120) pendant que l'appareil de laboratoire cible (120) est masqué.

7. Procédé selon l'une quelconque des revendications précédentes, comprenant en outre,
l'accès manuel aux portes-récipients d'échantillons d'essai (160) par un opérateur de laboratoire dans l'appareil de laboratoire cible (120) masqué pendant que l'appareil de laboratoire cible (120) est masqué.

8. Procédé selon l'une quelconque des revendications précédentes, dans lequel l'appareil de laboratoire cible (120) est un appareil d'archivage tel qu'un réfrigérateur.

9. Procédé selon l'une quelconque des revendications précédentes, dans lequel le masquage et le démasquage de l'appareil de laboratoire cible (120) est automatiquement déclenché par l'intergiciel de laboratoire (150) du système de laboratoire (100).

10. Procédé selon l'une quelconque des revendications précédentes, comprenant en outre,
le réacheminement des portes-récipients d'échantillons d'essai (160) de l'appareil cible (120) masqué vers d'autres appareils de laboratoire dans la pluralité d'appareils de laboratoire (130, 140) dans le système de laboratoire (100) pendant que l'appareil de laboratoire cible (120) est masqué.

11. Système de laboratoire (100), le système de laboratoire comprenant :
une pluralité d'appareils de laboratoire (115, 120, 130, 140),
au moins un tampon (170),
un système de transport (110), et
un intergiciel de laboratoire (150),
**caractérisé en ce que** le système de laboratoire (100) est configuré pour réaliser le procédé selon au moins l'une des revendications précédentes.
